Europäisches Patentamt

European Patent Office

Office européen des brevets

(11) Publication number: **0 348 174**
**A2**

# (12) EUROPEAN PATENT APPLICATION

(21) Application number: **89306264.6**

(22) Date of filing: **21.06.89**

(51) Int. Cl.⁴: **G 01 N 33/53**
**G 01 N 33/546**

(30) Priority: **23.06.88 US 210467**

(43) Date of publication of application:
**27.12.89 Bulletin 89/52**

(84) Designated Contracting States: **DE FR GB IT**

(71) Applicant: **BIO-RAD LABORATORIES, INC.**
**1000 Alfred Nobel Drive**
**Hercules California 94547 (US)**

(72) Inventor: **Murthy, Shashidhara H.M.**
**288 Shipley Avenue**
**Daly City California 94015 (US)**

**Watkins, Michael Irwin**
**133 Robin Court**
**Hercules California 94547 (US)**

(74) Representative: **Harrison, David Christopher et al**
**MEWBURN ELLIS 2 Cursitor Street**
**London EC4A 1BQ (GB)**

(54) Sperm antibody test.

(57) The present invention relates to compositions and methods for the detection of sperm antibodies bound to the surface of spermatozoa or present in human serum, seminal plasma or cervical mucous through the use of immunoassays employing latex beads coupled to anti-human immunoglobulins. Preferably the beads are monodispersed polystyrene beads coupled to the anti-human immunoglobulins through a bridge of a polyamine, such as polylysine, having amino acid residues of about 200 to about 1,000. The beads are also preferably about 2 to about 4 microns in size.

EP 0 348 174 A2

**Description**

## SPERM ANTIBODY TEST

The present invention relates to novel immunoassays for medical diagnoses and more specifically to diagnostic tests for the presence of sperm antibodies. The present invention provides methods for the detection of particular sperm antibodies bound to the surface of spermatozoa or present in human serum, seminal plasma or cervical mucous.

The presence of anti-sperm antibodies is associated with infertility in both men and women. Anti-sperm antibodies (also referred to herein as "sperm antibodies") vary in their impact on the reproductive capacity. For instance, sperm antibodies binding to the tail region of the spermatozoa may inhibit motility of the sperm and sperm antibodies binding to the head region of the spermatozoa may inhibit egg fertilization. Sperm antibodies of different classes (e.g., IgA, Igm, or IgG, etc.) may vary in their impact on the reproductive capacity because they are of different molecular weights, may have different numbers of antigen-reactive sites and may or may not interact with the complement system. Further, the presence of larger concentrations of sperm antibodies would be expected to have a greater impact on fertility. Several methodologies have been described to detect these immunoglobulins in serum and on sperm cells.

Previous tests for anti-sperm antibodies have largely relied on nonspecific antibody tests which are used to detect sperm antibodies in serum by complement-dependent immobilization reactions or by inducing agglutination. Tests using immobilization of spermatozoa are dependent upon immunoglobulin class and location of antibody binding upon the sperm surface. False negative results may occur with these tests because IgAs, for instance, do not fix complement and because many head-directed antibodies do not lead to loss of the motility of the sperm, although they may impair the egg-penetrating ability of the sperm.

Tests using agglutination where sperm cells are joined together, are dependent upon the spacing of antigen on the sperm surface to which specific antibodies are directed. These tests may produce inconsistent and unreliable results as the number of sites on the antibody molecule which combine with antigen varies with the immunoglobulin class, and large multivalent isotypes (IgMs and secretory IgAs) may be more likely to lead to cross-linking of sperm. Further, nonimmunoglobulin-mediated agglutination can be caused by bacteria and amorphous material within semen, as well as by nonimmunoglobulin proteins in serum, leading to false-positive reactions. See, Bronson et al, "Sperm antibodies: their role in infertility," Fertility and Sterility 42:171-183 (1984) for a summary of the various tests used to detect antibodies to spermatozoa.

Immunobeads, micron-sized polyacrylamide spheres, covalently bound to rabbit anti-human immunoglobulins have been used to directly detect antibodies of IgA, IgG, or IgM class on the surface of living sperm. The attachment of the immunobeads to the sperm cells is examined through the use of a phase contrast microscope. See Bronson et al, supra. Immunobeads linked to such anti-human antibodies have also been used to detect sperm antibodies in serum samples by first mixing antibody-free sperm with the serum and then placing the sperm-serum suspension in the immunobead suspension and examining the beads for attachment as above. Carson et al, "Antibody binding patterns in infertile males and females as detected by immunobead test, gel-agglutination test, and sperm immobilization test," Fertility and Sterility 49:487-492 (1988).

Use of the immunobeads in a sperm antibody assay has several disadvantages. Though the immunobeads are manufactured and sold in varying sizes, the present technology does not economically allow the production of uniform sizes. In general, 95% of the beads produced and available are less than 7 microns in size with no minimum size being described. This size variation may cause non-specific binding of antibodies to the beads by creating some physical interference with the binding process. Further, the larger beads may block the examination ability of an observer to determine the binding incidence. In addition, because of the optical properties of the beads a phase-contrast microscope is required for the examination of beads attached to spermatozoa.

Thus, simpler and more accurate methods for determining the presence of sperm antibodies in a human patient are needed. The present invention provides such novel methods.

## SUMMARY OF THE INVENTION

This invention provides methods and compositions for detection of sperm antibodies through the use of latex beads bound to anti-human immunoglobulins. Preferably the latex beads are polystyrene beads, and most preferably, they are carboxylate-modified polystyrene beads. Typically the beads are bound to the immunoglobulins through a bridge of a polyamine, such as polylysine, having amino acid residues of about 200 to about 1,000. The beads are also preferably about 2 to about 4 microns in size. One method for detecting sperm antibodies bound to the surface of spermatozoa comprises contacting sperm cells to the latex beads of the present invention, and examining the latex beads for attachment of the sperm cells to the latex beads.

Another method of the present invention provides for the detection of sperm antibodies in a human serum sample or in a cervical mucous sample comprising mixing sperm cells with a human serum or cervical mucous sample to form a suspension, contacting the suspension to the latex beads of the present invention, and examining the latex beads for attachment of the sperm cells to the latex beads.

DETAILED DESCRIPTION

The present invention provides novel methods for detecting sperm antibodies on the surface of spermatozoa by contacting sperm cells to latex beads which have been bound to anti-human immunoglobulins and examining the latex beads for attachment of the beads to the sperm cells. Alternatively, the present invention permits the detection of the presence of the sperm antibodies in a human serum sample by mixing sperm cells with a serum sample of interest and contacting the sperm-serum sample to latex beads which have been previously bound to anti-human immunoglobulins and then examining the beads for attachment of the sperm to the beads. Under the methods of the present invention, the attachment of the beads to the sperm may be determined with the use of a standard bright-field microscope.

Sperm antibodies, the target of the novel diagnostic procedures described herein, are those antibodies which are reactive with or capable of binding to sperm antigens of human origin. Most particularly the antibodies to be detected by this invention are capable of binding to surface antigens on spermatozoa.

The latex beads used in the present invention are synthetic water insoluble polymers which can be colloidally dispersed in aqueous solutions. They also contain one or more functional groups such as carboxyl, amine, hydroxyl, sulfhydryl, nitro, amide groups or the like. Preferably the latex polymers are monodispersed and will be carboxylate-modified polystyrene particles to accommodate binding of the particles to free amine or carboxyl groups present on an immunoglobulin. Vinyl acids, acrylic, itaconic, fumaric and other vinyl carboxylic acids may be copolymerized with styrene. The latex beads are preferably sphere-like in shape and range in size from about 0.2 to about 8 microns, preferably about 2 to about 4 microns in size, and are most preferably about 3 microns in size. Typically the latex beads will be uniform in size with a standard deviation of about 10% or less, most preferably about 5% or less, of the diameter of the particle. Examples of such beads are available and may be obtained from Polyscience, Warrington, PA; Seragen, Indianapolis, IN; or Duke Scientific, Palo Alto, CA.

Typically a bridge, spacer arm or other linking moiety is first coupled to the latex bead which increases the functional groups available for attachment of the bead to an antibody. Such bridges may include polyamines such as polylysine, polyornithine, polyasparagine or polyarginine, for example. These may be obtained from Sigma Chemical Co., St. Louis, MO. Typically these polyamines will have amino acid residues of at least 2 to about 5000, preferably about 200 to about 1000, and most preferably about 400 to about 500. Poly-L-lysine (MW 49,000) is the most preferred of such linkers. The polyamines and the latex beads are mixed together. The bridge is then coupled to the latex bead using carboxyl activating reagents. Typically one uses an aromatic or aliphatic carbodiimide as a carboxyl activating reagent. The carboxyl activating reaction takes place using approximately 2 to 20 molar excess of a carboxyl activating reagent such as 1-ethyl-3-(3-diethylaminopropyl) carbodiimide (EDAC), which is preferred, in an aqueous buffer such as phosphate buffered saline containing 0.01% Tween 20 at pH 5.5 to 7.0 for about 18 hours at 2°C to 10°C. The excess and unreacted carboxyl activating reagent may then be removed by centrifugation or filtration of the beads. One process for forming an amide linkage between an antibody and latex is described in U.S. Patent No. 4,045,384 issued August 30, 1977, which is incorporated by reference herein.

The latex beads, preferably coupled to a linker such as polylysine, are bound to anti human immunoglobulins. The term "anti-human immunoglobulin(s)" includes polyclonal antibodies, monoclonal antibodies, reactive fragments thereof, such as Fv, Fab, F(ab)$_2$, synthetic immunoglobulins and recombinant immunoglobulins including chimeric immunoglobulins or their derivatives and a combination of any of the above which are reactive with or capable of binding to human antibodies. Preferably these immunoglobulins are of the Igm, IgG or IgA class or comprise a combination of one or more of those classes. Further, such immunoglobulins may be of mammalian origin, preferably of goat, rabbit, murine or human origin. Sources for anti-human antibodies are well-known in the art, as well as the methods for producing them. The anti-human immunoglobulins are typically linked to the latex beads through the use of a carboxyl activator, such as EDAC, as described above.

Binding reactivity between antibodies and antigens is well understood in the art. For this invention, an anti-human immunoglobulin is reactive with a human antibody when the anti-human immunoglobulin coupled to a latex bead is capable of attaching a human antibody to the latex bead under the buffer systems described herein.

The latex beads are then typically suspended in a bead buffer solution with a pH of about 5.0 to about 8.0, preferably of about 7.0 to about 8.0, and most preferably to about 7.4. The bead buffer may be any nontoxic buffer generally used in immunoassays such as Tris-HCl, or Hepes, more preferably, a phosphate buffer. The buffer may also contain salts at physiologically acceptable levels such as magnesium chloride, potassium chloride, sodium chloride, sodium pyruvate, calcium chloride or the like to maintain the tonicity of the sperm. Preservatives such as antibiotics, streptomycin, penicillin or kanamycin or p-hydroxybenzoic acid methyl ester may be added to promote the longevity and usefulness of the buffer. Simple sugars as energy sources to promote sperm motility may be added as, for example, D-glucose at concentrations of about 500 to about 1500 mg/l. Bovine Serum Albumin (BSA) at concentrations of about 2.5 to 7.5 g/l may also be added to simulate the natural sperm environment. The bead buffer may also contain surfactants in low concentrations of about 0.005 to about 0.05% such as Brij 35 (Sigma Chemical Co.), Titron X-100 (Sigma Chemical Co.), Tween 20 (Bio-Rad Laboratories, Hercules, California); or sodium dodecylsulfate. A dispersant agent, for example polyethylene glycol (PEG), is also preferably added to between about 5 to about 20 g/l. Most preferably, if PEG is used, PEG having an average molecular weight of about 7000 to about 9000 should be chosen for the suspension. Though not critical, about 12.5 g/l of beads in the buffer solution is preferred.

The latex beads as described above may be used in a variety of procedures to assay for the presence of sperm antibodies. The latex beads may be used to determine the presence of sperm antibodies which are present directly on the sperm. For optimal results active semen or seminal plasma samples of interest should be assayed within 2 to 4 hours of collection and should be maintained at about 37°C until used. Frozen sperm samples, though less preferable, may also be used. Such samples may be frozen by any of the methods well-known in the art for maintaining viable sperm.

The semen sample is first liquified by, for example, gentle heating to 37°C for about 20 minutes. The sperm sample is then washed with the wash buffer by sequential low speed centrifugations. The final suspension of sperm to be placed in contact with the latex beads should comprise 12% or more motile sperm and should have a concentration of about $10 \times 10^6$ to about $300 \times 10^6$, preferably $20 \times 10^6$ to about $200 \times 10^6$, most preferably about 50 to $100 \times 10^6$ motile sperm/ml of sample.

Typically the semen or seminal plasma sample to be tested is washed in a wash buffer solution with a pH adjusted as described above for the bead buffer. The wash buffer is similar to the bead buffer and is preferably a standard phosphate buffer. Preferably the wash buffer will contain some of the ingredients as described above for the bead buffer, although a surfactant or a dispersant agent is not typically used for the wash buffer. In addition, a pH indicator, such as phenol red may be added.

For direct assay of antibodies bound to the surface of spermatozoa the sperm cells are contacted with the latex beads bound to anti human immunoglobulins by mixing the sperm suspension with the bead suspension in a ratio of about 0.5 to 1.5 to about 5 to 15, preferably 0.8 to 1.2 to about 9 to 12, and most preferably about 1 to about 10 to determine the presence of sperm antibodies on the sperm in a sample. The ratio of the sperm suspension to the bead suspension will vary depending on the concentration of motile sperm and the concentration of beads in the bead suspension. One skilled in the art would readily be able to determine an effective ratio of the two suspensions depending on the conditions present. The sperm-bead suspension should be examined within 30 minutes after mixing.

The sperm-bead suspension, or a portion thereof, is then examined to determine whether or not any of the spermatozoa have attached to a latex bead. This may be most simply and most preferably done through the aid of a magnifying device such as a bright-field microscope, a phase contrast microscope, or the like. Alternatively, other methods known in the art may be used for detecting antibody binding activity such as the use of fluorescein-conjugated antiglobulins or radiolabeled antiglobulins as the anti-human immunoglobulins. If spermatozoa have attached to the latex beads, then the presence of sperm antibodies is indicated.

The location of the attachment of the sperm to the latex beads may be determined by physically examining at what point on the sperm the sperm has been attached to the bead by the sperm antibody. For instance, it may be useful to know if the sperm antibodies are binding to the head, tail or middle part of the sperm. This yields some information regarding the type of sperm antibodies present and what effect they may have on the activity of the sperm. In addition, selective use of classes or subclasses of anti-human immunoglobulins may permit one to determine what class of sperm antibodies are present. For example, if IgA anti-human antibodies are linked to the latex beads, an assay may be run to determine whether IgA sperm antibodies are present. Separate assays may be run for separate classes or subclasses of antibodies.

As an alternative to the above-described direct methods, an indirect assay for determining the presence of sperm antibodies in serum samples may also be run by utilizing the latex beads coupled to anti-human immunoglobulins. To conduct the indirect assay, human serum samples of interest are obtained and the complement of the serum sample should be inactivated. Typically this is done by incubating the serum at an elevated temperature of about 56°C for about 30 minutes. The serum sample should be diluted with a wash buffer such as one of those described above, preferably in a ratio of serum to buffer of about 1 to about 3.

The serum sample is then incubated with a sperm sample known to be free of sperm antibodies. The ratio of the two samples will vary depending on the concentration of motile sperm and the titer of any sperm antibodies which may be present. One skilled in the art will readily be able to determine an appropriate ratio. Incubation of the serum sample with the antibody-free sperm should occur for a period of time sufficient to allow for binding of any sperm antibodies present in the serum to the sperm, typically, from about 10 to about 60 minutes at about 37°C. The serum sample used in this assay may be of male or female origin.

Such an antibody-free semen sample may be obtained by the methods described herein, or by any of the methods described and known in the art such as those described in Bronson et al, supra. The semen sample is washed and liquified, as described above, prior to incubation and contact with the serum sample. The antibody-free sperm sample should have motile sperm of 50% or more, preferably 60% or more, most preferably 70% or more. In addition, the sperm sample should have a final count of about $10 \times 10^6$ to about $300 \times 10^6$, preferably $20 \times 10^6$ to about $200 \times 10^6$, most preferably $50 \times 10^6$ to about $100 \times 10^6$ motile sperm/ml prior to incubation with the serum sample.

The sperm-serum suspension is contacted with the latex beads bound to anti-human immunoglobulins by mixing the sperm-serum suspension with the bead suspension, described above, such that the ratio of the sperm-serum suspension to the bead suspension ranges from about 0.5 to 1.5 to about 5 to 15, preferably 0.8 to 1.2 to about 9 to 12, and most preferably 1 to 10. The ratio of the two suspensions will vary depending on the concentration of motile sperm, the titer of any sperm antibodies which may be present in the serum and the concentration of beads in the bead suspension. One skilled in the art would readily be able to determine an effective ratio of the two suspensions depending on the conditions present.

The sperm-serum-bead suspension, or a portion thereof, is then examined within 30 minutes to determine whether or not any of the spermatozoa have attached to a latex bead. This may be done by any of the methods described above in connection with the direct method. If spermatozoa have attached to the latex beads, then the presence of sperm antibodies in the serum sample is indicated. The location of the attachment of the sperm to the latex beads may also be observed and recorded as described above. Further, selective use of subclasses of anti-human immunoglobulin, as described above, may permit one to determine what class of sperm antibodies are present in the serum.

Further, a cervical mucous sample taken from a female patient may be tested for the presence of sperm antibodies in a manner similar to the test of the serum samples as described above by substituting the mucous sample for the serum sample. The cervical mucous sample, however, should first be liquefied through the use of an enzyme such as Bromelin (Sigma) or other procedures known in the art.

Kits to test for the presence of sperm antibodies are also contemplated by this invention. Kits would include vials of latex beads coupled to anti-human immunoglobulins. The kits could further comprise vials of latex beads coupled to anti-human immunoglobulins by a bridge of poly-L-lysine. The kits could also include vials of wash buffer, and control sera (preferably lyophilized). One of the vials of the control sera would be positive for sperm antibodies and one of the vials of the control sera would be negative for sperm antibodies. The kit would also contain an instruction sheet for using the kit contents to assay for sperm antibodies.


EXAMPLES


Example I - Direct Method

Using this invention one can determine the presence or absence of sperm antibodies found on the sperm cells in a semen sample.

1. Preparation of the Latex Beads.

Latex beads of a 3μ size (Duke Scientific) were coupled to poly-L-lysine (MW 49,000) using 1 ethyl-3-(3-dimethylaminopropyl) carbodiimide (EDAC).

Two grams of latex beads were suspended in a tube containing 40 ml of 50 mM of phosphate-buffered saline (PBS), pH 6.2 containing 0.01% Tween 20. Four hundred mg of poly-L-lysine was added. Then 160 mg of EDAC was weighed out and added. The tube was immediately inverted several times to mix and then placed on a rocker for 18 hours at 4°C. The suspension was then centrifuged and the supernatant was carefully decanted and discarded. The pellet was resuspended, centrifuged and decanted two more times. The last pellet was resuspended in 35 ml of 50 mM PBS, pH 6.2 containing 0.01% Tween 20. Aliquots of that suspension were placed into three tubes labeled IgA, IgG and IgM in a ratio of 2:2:1 by volume, respectively.

Human anti-Igm, anti-IgG, and anti-IgA immunoglobulins were obtained from Dako Corporation, Santa Barbara, CA. To the IgA tube were added 32 mg of anti-IgA immunoglobulins, to the IgG tube were added 32 mg of anti-IgG immunoglobulins, and to the IgM tube were added 16 mg of anti-IgM immunoglobulins. These three tubes were then inverted several times to mix. EDAC was then quickly weighed out so that 96 mg of EDAC were added to both the IgA and the IgG tube and 48 mg of EDAC were added to the IgM tube. After each addition of EDAC the tube was immediately inverted several times to mix. The tubes were then all placed on a rocker for 18 hours at 4°C.

At the end of 18 hours, the tubes were centrifuged and the supernatant was decanted off and discarded. The pellets were each resuspended in 35 ml of 50 mM PBS, pH 6.2 containing 0.01% Tween 20. Once again the tubes were centrifuged and the supernatant decanted. The pellets were each resuspended, centrifuged and decanted two more times. The final pellet was resuspended in 15 ml of bead buffer (described below), centrifuged and decanted. This pellet was then resuspended in bead buffer, centrifuged and decanted two more times. The IgA and IgG tubes were brought to a volume of 64 ml with bead buffer and the IgM suspension was brought to a volume of 32 ml with the bead buffer. The three solutions were mixed to give a final bead concentration of 12.5 mg/ml and a ratio of IgA:IgG:IgM of 2:2:1 by volume, respectively.

The bead buffer contained the following components:

| Substance | Grams/Liter |
|---|---|
| Magnesium Chloride 6H$_2$O | 0.100 |
| Potassium Chloride | 0.200 |
| Potassium Phosphate Monobasic | 0.200 |
| Sodium Chloride | 8.000 |
| Sodium Phosphate Dibasic (Anhydrous) | 1.150 |
| Sodium Pyruvate | 0.036 |
| Streptomycin Sulfate | 0.050 |
| Kanamycin Monosulfate | 0.100 |
| D-Glucose | 1.000 |
| Calcium Chloride 2H$_2$O | 0.133 |
| Bovine Serum Albumin | 5.000 |
| p-Hydroxybenzoic Acid Methyl Ester | 100 mg |
| Polyethylene Glycol 8000 | 15.000 |
| Brij 35 | 100 mg |

The pH of the buffer was adjusted to 7.4 with HCl.

3. Semen Sample.

Semen samples were obtained from three patients, DO, ZE, and RA who were suspected of having sperm antibodies. These semen samples had a motility of 50% or more and a sperm count of greater than $10^6$ motile sperm per ml of semen sample. Semen samples were tested within 2 hours of collection and were maintained at 37°C until used.

4. Wash Buffer for Semen.

A wash buffer was prepared for washing the sperm samples and contained the following components:

| Substance | Grams/Liter |
|---|---|
| Magnesium Chloride 6H$_2$O | 0.100 |
| Potassium Chloride | 0.200 |
| Potassium Phosphate Monobasic | 0.200 |
| Sodium Chloride | 8.000 |
| Sodium Phosphate Dibasic (Anhydrous) | 1.150 |
| Sodium Pyruvate | 0.036 |
| Streptomycin Sulfate | 0.050 |
| Penicillin-G Sodium | $10^6$ units |
| D-Glucose | 1.000 |
| Calcium Chloride 2H$_2$O | 0.133 |
| Phenol Red | 20 mg |
| p-Hydroxybenzoic Acid Methyl Ester | 100 mg |
| Bovine Serum Albumin | 5.000 |
| Sodium Bicarbonate | 2.1 g |

The pH was adjusted to 7.1 with HCl. The buffer was then heated to 37°C with cap securely in place before use.

5. Assay Procedure.

The semen sample was prepared by heating in a sealed container for 20 minutes at 37°C. Wash buffer, twice the volume of the semen sample, was added and mixed. The sample was then centrifuged at 500-600g for 5 minutes. The supernatant was pipetted off and the pellet was washed and centrifuged, as above, 2 to 4 more times using 3 ml of wash buffer each time. The final pellet was resuspended in wash buffer to give a concentration of 50 to 100 x $10^6$ motile sperm/ml. Five μl of the sperm suspension were added to 50 μl of the prepared bead suspension in a clean test tube and this suspension was mixed well.

About 20 μl of the sperm-bead suspension was placed on a glass slide with a coverslip. The slide was examined immediately under a phase contrast microscope at 400x. The proportion of 100 motile sperm counted with beads bound to their surface was determined. The location of the bound beads was also recorded in terms of whether the beads bound to the head, midpiece, or tail of the sperm. Sperm with beads bound at any two locations were recorded as having beads bound to the entire sperm. Those sperm labelled as "free sperm" were those which did not bind to any beads. The results for the three patients are set forth below in Table 1:

6

TABLE 1

| Sample | %Free | %Tail | %Head | %Midpiece | %Entire |
|---|---|---|---|---|---|
| Negative | 100 | | | | |
| Positive | 58 | 29 | | | 13 |
| DO | 100 | | | | |
| ZE | 81 | 19 | | | |
| RA | 45 | 49 | 6 | | |

No antibodies were present in the negative control or in the sample from patient DO as the sperm sample demonstrated no binding to the beads in each case. The positive control, however, demonstrated binding to the tail and entire regions to a total of 42% of the sperm. Patient ZE had a sperm sample wherein 19% of the sperm bound at the tail region to the beads and patient RA had a sperm sample wherein a total of 55% of the sperm bound to the beads at the head and tail regions.

Example II - Indirect Protocol

1. Preparation of the Semen Sample.
A semen sample was obtained which was previously determined to be anti-sperm antibody negative. The sample was prepared as above by heating to 37°C for 20 minutes in a sealed container. The wash buffer, described above, was added to twice the volume of the semen sample and the sample was mixed. The semen-buffer solution was centrifuged for 5 minutes at 500-600g. The supernatant was pipetted off and the pellet was washed and centrifuged two more times using 3 ml of wash buffer each time. The final pellet was resuspended in wash buffer to give a concentration of 50 to 100 x $10^6$ motile sperm/ml.

2. Preparation of the control sera.
Lyophilized 2 ml serum samples known to be positive for sperm antibodies and sperm samples known to be negative for sperm antibodies were used as controls. To each of the control samples were added 4 ml of deionized water. The sample bottles were inverted several times to mix. The samples were allowed to let stand at room temperature until they were completely dissolved.

3. Preparation of the Serum-Sperm Sample.
Serum samples were obtained from patients, WC, PD, and PS. The complement was inactivated by incubating at 56°C for 30 minutes. In a container, 400 µl of each serum sample was mixed with 1200 µl of the wash buffer, described above. Then, 100 µl of the sperm suspension from step 1 above, was added. The serum-sperm suspension was incubated at 37°C for 30 minutes to allow for binding of any anti-sperm antibodies in the serum to the normal sperm. After incubation, 3 ml of the wash buffer was added and mixed. This mixture was centrifuged at 500-600g for 5 minutes. The supernatant was pipetted off and the wash and centrifugation step was repeated two more times.

4. Assay Procedure.
After the final centrifugation in step 2, 100 µl of the supernatant was left behind to suspend the sperm and 5 µl of the sperm suspension was added in a clean test tube to 50 µl of the bead suspension, prepared as in Example I. The sperm-bead suspension was mixed well and then 20 µl of the suspension was placed on a glass slide and a coverslip was placed on top. The slide was examined immediately under phase contrast at 400x for sperm binding to the beads as described in Example 1, above. The results for the binding locations and the presence of sperm antibodies using this indirect method are shown in Table 2 as follows:

TABLE 2

| Sample | %Free | %Tail | %Head | %Midpiece | %Entire |
|---|---|---|---|---|---|
| Negative Control | 100 | | | | |
| Positive Control | 25 | 63 | 4 | | 8 |
| WC | 18 | 34 | 10 | | 38 |
| PD | 74 | 22 | | | 4 |
| PS | 32 | 36 | 14 | 2 | 16 |

As can be seen from the Table, the negative control demonstrated no binding of the sperm to the beads and the positive control demonstrated 75% of the sperm binding to the beads at the tail, head and entire locations. The serum sample from patient WC demonstrated binding of 82% of the sperm to the beads at the tail, head and entire locations; the serum sample from patient PD demonstrated binding of 26% of the sperm to the beads at the tail and entire locations; and the serum sample from patient PS demonstrated binding of 68% of the sperm to the beads at the tail, head, midpiece and entire locations.

Example III - Preparation of Cervical Mucous Samples

Prepare a 2 mg/ml solution of Bromelin in wash buffer, prepared as in Example I. Mix together equal volumes of Bromelin solution and the cervical mucous sample. Incubate at room temperature for 10 minutes, shaking every few minutes. Test liquefication by drawing the solution into a pipette and observing the drops expelled. Centrifuge the mixture at 600 g for 10 minutes to sediment cells and debris. Heat-inactivate the cervical mucous supernatant by heating to 56°C for 30 minutes. Add 5 x $10^6$ antibody-free spermatozoa directly to 200 $\mu$l of cervical mucous supernatant. Incubate the sample at 37°C for 60 minutes, and then transfer it to a 15 ml centrifuge tube. Add 10 ml of wash buffer and centrifuge the sample at 800 g for 8 minutes. Decant the supernatant and repeat one more time. Afterwards, resuspend the pellet in 500 $\mu$l of wash buffer. Microfuge sample for 8 seconds. Discard supernatant and resuspend pellet in 50 $\mu$l of wash buffer. Mix 5 $\mu$l of this suspension with 50 $\mu$l of bead suspension, prepared as in Example I. Mix and add 20 $\mu$l to a microscope slide, cover with a coverslip and examine under a microscope.

Although the present invention has been described in some detail by way of illustration and example for purposes of clarity and understanding, the illustrations and examples should not be construed as a limitation upon the claims.

**Claims**

1. A method for detecting sperm antibodies bound to the surface of spermatozoa comprising:
   (a) contacting sperm cells to latex beads bound to anti-human immunoglobulins through a bridge, and
   (b) examining said latex beads for attachment of said sperm cells to said latex beads.

2. A method according to claim 1, wherein the bridge is a polyamine having amino acid residues of about 200 to about 1,000.

3. A method according to claim 1, wherein the bridge is a polylysine having amino acid residues of about 400 to about 500.

4. A method according to claim 1, wherein the latex beads are bound to the immunoglobulins through a bridge of poly-L-lysine.

5. The method of claim 1, wherein the latex beads are about 2 to about 4 microns in size.

6. A composition comprising a latex bead bound to anti-human immunoglobulins through a bridge of a polylysine having amino acid residues of about 200 to about 1,000.

7. A composition according to claim 6, wherein the bridge is poly-L-lysine.

8. A composition according to claim 6, wherein the latex beads are about 2 to about 4 microns in size.

9. A method for detecting sperm antibodies bound to the surface of spermatozoa comprising:
   (a) contacting sperm cells to latex beads of about 2 to about 4 microns in size bound to anti-human immunoglobulins, and
   (b) examining the latex beads for attachment of the sperm cells to the latex beads.

10. The method of claim 9, wherein the latex beads are bound to the anti-human immunoglobulins through a polyamine bridge having amino acid residues of about 200 to about 1,000.